# EUROPEAN PATENT SPECIFICATION

(11) **EP 1 787 569 B1**
(45) Date of publication and mention of the grant of the patent: **12.01.2011**
(21) Application number: 05024985.3
(22) Date of filing: 16.11.2005
(51) Int. Cl.: A47L 15/50, A61L 2/26

(54) **Storing device for a dishwashing machine**
Lagervorrichtung für eine Geschirrspülmaschine
Dispositif de stockage pour une lave-vaisselle

(43) Date of publication of application: 23.05.2007
(73) Proprietor: Electrolux Home Products Corporation N.V., 1930 Zaventem (BE)
(72) Inventor: Amaral, Henrique, 90763 Fürth (DE); Westergard, Stefan, 21047 Saronno VA (IT); Hofmann, Hartmut, 90518 Altdorf (DE); Salmaso, Daniela, 33170 Pordenone (IT); Chrestenzen, Lizandro, 33170 Pordenone (IT); Salerno, Luigi, 20092 Cinisello B. Milano (IT)
(74) Representative: Hochmuth, Jürgen

(56) References cited:
- DE-A1- 2 755 892
- DE-A1- 2 946 591
- DE-A1- 10 311 888
- DE-U1- 20 304 305
- FR-A- 2 021 050
- US-A- 3 025 864
- US-A- 3 266 632
- US-A- 3 289 683
- US-A- 4 593 822
- US-A- 4 917 248
- US-A- 6 048 504
- PATENT ABSTRACTS OF JAPAN vol. 2000, no. 03, 30 March 2000 (2000-03-30) & JP 11 332809 A (TOTO LTD), 7 December 1999 (1999-12-07)
- PATENT ABSTRACTS OF JAPAN vol. 2003, no. 11, 5 November 2003 (2003-11-05) & JP 2003 190071 A (MATSUSHITA ELECTRIC IND CO LTD), 8 July 2003 (2003-07-08)
- PATENT ABSTRACTS OF JAPAN vol. 1998, no. 03, 27 February 1998 (1998-02-27) & JP 09 285435 A (HITACHI LTD), 4 November 1997 (1997-11-04)

## Description

The present invention relates to an auxiliary storing device for storing small to medium sized cutlery, dishes and/or glassware in or on a basket of a dishwashing machine.

In a conventional dishwashing machine cutlery is stored in a cutlery basket placed in the lower basket of the dishwashing machine or on a separate tray formed as top drawer of the dishwashing machine. Delicate glassware is stored on inclined glass supports at the upper basket. When loading cutlery, dishes and glassware into the dishwashing machine, the individual parts are sorted and placed at the convenient position or compartment of the dishwashing machine.

An auxiliary storing device for a basket of a dishwashing machine which provides a conventional basket with a quick access loading zone is known from JP 11 332 809 A. However, this known device has the problem that delicate items can be damaged if the storing device tips, for instance due to the weight of the said items placed on it.

The invention is defined in claim 1, and solves the above-mentioned problem.

Particular embodiments are set out in the dependent claims.

For normal loading processes for loading cutlery and dishes into a dishwashing machine the user needs some time to sort the cutlery and dishes and to place them at the appropriate location on the upper or lower basket, for example cutlery in the cutlery basket on the lower basket, glasses on glass supports at the side of the upper basket, and small and large plates in the corresponding plate inserts in the lower or upper basket. This is alright, when the user has enough time and when all users take care while loading. Time however is sometimes limited and last minute placements are then not made so carefully. In particular small children only partially open the dishwashing machine, partially draw out the upper basket and place their dishes anywhere in the front region of the basket. The invention provides a mat device or flexible or soft coated shelf having a quick access zone for last minute loading of the dishwashing machine. In the present context, a dish or dishes which can be placed on the auxiliary storing device of the invention preferably comprise(s) any plate, bowl, cup, pot, jug, pitcher, vase etc. which is made from any suitable material such as e.g china, earthenware, any plastics material or the like, as well as glassware of any kind which can be places on the auxiliary storing device of the invention such as e. g. a drinking glass of any kind, a glass plate, glass bowl, glass cup, glass pot, glass jug, glass pitcher, glass vase etc..

The device of the present invention can be arranged in a basket of a dishwashing machine, preferentially on the bottom of the basket and is defined by independent claim 1. The flexible mat device has a base structure adapting at least in part flexibly or elastically to the contour of the bottom of the basket or to the contour of the place in the basket where the flexible mat device is placed. Depending on the need, the flexible mat device may be placed into the basket or removed from it. Due to its flexibility and in consequence due to its at least partial adaptation to the contour of the basket, the flexible mat device can be placed nearly at all desired portions within the basket, and on the other hand the volume required within the basket for placing the mat device is minimal. When it is removed, the flexible mat device may be bent, e.g. folded or rolled and stored outside the dishwashing machine with minimum storing volume requirements. Due to its flexibility the flexible mat device is at least partially elastic, so that it is bent,e. g. it gives way elastically, when cutlery or dishes are placed on top of the flexible mat device preventing damage to delicate dishes e.g. delicate glassware or additionally adapting to the form of the cutlery or dishes placed on the flexible mat device resulting in an improved support within the basket.

According to the invention the flexible mat device is made of an elastic material or it is at least partially coated with an elastic material, in particular with a soft material, such that damages on dishes, e.g. glassware are further reduced. Also lateral movements on the flexible mat device are inhibited while the basket is drawn out of or pushed into the dishwashing machine. In particular, if dishes or cutlery are loaded in last minute in a rush movement the dropping of the dishes is dampened thereby avoiding mechanical stress to the dishes. Further, the generation of noise while loading/unloading the dishes or cutlery and during the dishwashing program or the movement of the basket is reduced.

The mat device of the invention comprises a rigid supporting structure. The supporting structure is at least partially covered by an elastic material in particular a soft material and/or a flexible structure in particular a soft flexible structure is attached thereto on which dishes or cutlery can be placed. The rigid supporting structure, which at least partially forms the base of the mat device, can at least partially bridge or span steps or unevenness in a basket of a dishwashing machine. For example one end of the mat device may be placed against a side wall of the basket, while the other end rests on the bottom of the basket. Then the mat device is inclined within the basket and the dishes or cutlery are placed on the soft elastic material or on the flexible structure in the quick access zone of the mat device. In an embodiment, rigid supporting structure portions of the mat device are linked with flexible connection elements, such that also the mat device having a rigid supporting structure may be bent and partially adapted to the contour of the basket, depending on its orientation within the basket.

It should be noted that the soft pad, i. e. the mat device of the invention may also be used as a shelf or as a supporting pad either of which provides the function of supporting dishes and cutlery and may optionally be placed within a basket of a conventional dishwashing machine at the desired location.

In a preferred embodiment a mat device comprises a flexible structure which comprises a plurality of supporting elements such as pins which are protruding from the base structure of the mat device of the invention and give an additional support for the cutlery and/or dishes placed on the mat device. Preferentially, the supporting elements are flexible or elastic, such that they are bent, when dishes like cups are placed on the mat device, thereby improving the mechanical stability of the placed goods. In a preferred embodiment, the supporting elements are made of two components, a soft component for example on the surface on which the goods are placed, and a rigid component on the bottom side or in the core of the supporting elements. Additionally or alternatively the supporting elements may be made of a semi-rigid material. In this case, the supporting elements are partially bent to a certain degree resulting in an adaptation to the contour of the goods placed on the mat device, while the semi-rigid supporting elements themselves exert a counter force onto the placed good.

In a further preferred embodiment, the supporting elements are at least partially hollow, such that their flexibility is improved. On the other hand, the mat device may be positioned onto a pin, for example a pin of a plate insert, by inserting its tip into the recess of the supporting element. Thereby the mat device may be mounted to the pins of the basket or may be spanned between pins of the basket.

According to the invention, the mat device has at least one mounting element for mounting the mat device on or in the basket, such that the mat device can be positioned in the basket in a desired orientation and/or the mat device is secured against sliding within the basket during its movement.

In a particularly preferred embodiment, the mat device can be clipped to at least one wire of the dishwasher basket.

In a further preferred embodiment, the mat device can simply be laid on the grid of the dishwasher basket.

Preferably, the ground area of the flat mat device is significantly smaller than the ground area of the basket where the mat device is to be placed. For example, the area is smaller than one third of the basket's area, preferentially smaller than one forth or one fifth of this area.

Reference is made in detail to a preferred embodiment of the invention, an example of which is illustrated in the accompanying drawings which show:
- Fig. 1: a perspective view on a soft pad, and
- Fig. 2: the soft pad of Fig. 1 placed within a basket of a dishwashing machine.

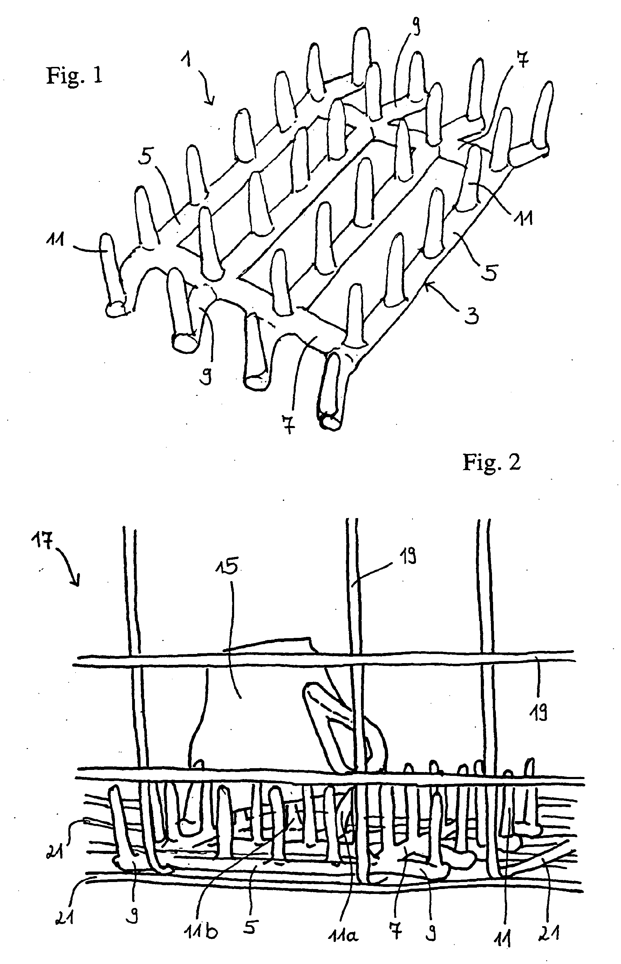

Fig. 1 shows a perspective view on a soft pad 1 which is homogenously fabricated from a single elastic material by injection molding. The elastic material provides flexibility to adapt and change the basic form of the soft pad and results in an elastic surface of the soft pad in the areas where cutlery or dishes are placed. Further, the elastic material is resistant against the temperature and chemical conditions used during the dishwashing cycles. On the other hand, the soft pad has a residual rigidity, such that it at least partially stretches back to its base form as shown in Fig. 1, when no force is exerted to the soft pad 1.

The soft pad 1 comprises a base frame 3 formed of longitudinal bars 5 and cross bars 7, thereby forming an interlinked platform. The longitudinal bars 5 are extending beyond the cross bars 7 and are curved downward to bends 9. When placed in a basket, the downward bending bends 9 mesh with the grid of the basket, thereby laterally fixing the soft pad 1 within a basket 17 as shown in Fig. 2.

Perpendicular to the upper surface of the longitudinal bars 5 and cross bars 7 flexible fingers (11) extend upward for receiving and supporting dishes and cutlery placed upon the soft pad 1.

Fig. 2 schematically shows the soft pad 1 of Fig. 1 placed within the basket 17 (only partially shown). The grid of the basket 17 is formed of side bars 19 and bottom bars 21. The soft pad 1 is placed here at the center front section of the upper basket 17. A cup 15 is placed on the soft pad 1 where the rim of the cup 15 is placed between the flexible fingers. Where the rim of the cup 15 runs over the center of fingers 11, the fingers are bent by the cup 15 and abut against the side or inner wall of the cup, as for example shown by fingers 11a and 11b.

The soft pad placed in the front section of the upper basket of a dishwashing machine provides a quick access zone for the user. Even if the washing machine's door is only partially opened and the upper basket is only partially drawn out of the tub, the user may place the dishes and cutlery even in a hastily manner upon the soft pad 1 without damage due to its soft and elastic bearing surface.

### Reference Numerals List

- 1: soft pad
- 3: base frame
- 5: longitudinal bar
- 7: cross bar
- 9: bend
- 11,11a,11b: flexible finger
- 15: cup
- 17: basket
- 19: side bar
- 21: bottom bar

## Claims

1. Mat device adapted to be arranged in a basket (17), in particular on the bottom of a basket (17), of a dishwashing machine, the mat device comprising a base structure (3) at least partially comprising a rigid supporting structure which is at least partially covered by an elastic material and/or supports a flexible structure (11) for receiving dishes (15) or cutlery wherein a plurality of supporting elements (11) are protruding from the base structure (3),
**characterized in that**
the mat device comprises at least one mounting element for mounting the mat device (1) in the basket (17), in particular on the bottom of the basket (17),

2. Mat device according to claim 1, wherein the mat device is secured against sliding within the basket during its movement.

3. Mat device according to claim 1 or 2, wherein at least one mounting element comprises at least one detent means, in particular at least one means for clipping the mat device to a wire of a basket (17) of a dishwashing machine.

4. Mat device according to any of claims 1 to 3, wherein at least one mounting element is arranged at the base structure (3) adapted to mount the mat device (1) on the bottom of a basket (17).

5. Mat device according to any of claims 1 to 4, wherein the supporting elements (11) comprise pins, studs, fingers, plate inserts and/or cup inserts.

6. Mat device according to any of claims 1 to 5, wherein the supporting elements (11) are flexible and/or elastic.

7. Mat device according to any of claims 1 to 6, wherein the supporting elements (11) comprise a soft and/or a rigid and/or a semi-rigid material.

8. Mat device according to any of claims 1 to 7, wherein the supporting elements (11) comprise two components including at least a soft component.

9. Mat device according to any of claims 1 to 8, wherein at least a portion of the supporting elements (11) are at least partially hollow, in particular at least a portion of the supporting element has a recess from below.

10. Mat device according to any of the previous claims,
wherein the base structure (3) has a web-shaped form.

11. Mat device according to any of the previous claims,
wherein the base structure (3) has flexible connecting elements (7, 9) which are interconnectable or interlinkable joining elements of the base structure (3).

12. Mat device according to claim 11, wherein the joining elements are node elements and each flexible connecting element (7, 9) interlinks at least two node elements.

13. Mat device according to any of the previous claims,
wherein the mat device (1) is at least partially formed of elastic material.

14. Mat device according to claim 13 as depending on claim 1, wherein at least the base structure (3) is formed of elastic material.

15. Mat device according to any of the previous claims,
wherein the base structure (3) and/or the supporting elements (11) are composed of a flexible material and/or a semi-rigid material.

16. Mat device according any of the previous claims,
wherein the flexible material is EPDM, Sandoprene or a soft plastic material.

17. Mat device according to claim 15 or 16, wherein the semi-rigid material is a thin metal wire or a plastic material, in particular being coated with an elastic material.

18. Mat device according to any of the previous claims,
wherein the width of the mat device (1) is less than 30 cm, in particular less than 25 cm, preferentially less than or equal to 20 cm.

19. Mat device according to any of the previous claims,
wherein the depth of the mat device (1) is less than 30 cm, in particular less than 25 cm, preferentially less than or equal to 20 cm.

20. Dishwashing machine or basket (17) of a dishwashing machine comprising a mat device (1) according to any of the previous claims.

## Patentansprüche

1. Mattenvorrichtung, die zur Anordnung in einem Korb (17), insbesondere am Boden eines Korbes (17) einer Geschirrspülmaschine geeignet ist, wobei die Mattenvorrichtung eine Basisstruktur (3) umfasst, die wenigstens teilweise eine steife Trägerstruktur umfasst, die wenigstens teilweise von einem elastischen Material bedeckt ist und/oder eine flexible Struktur (11) zur Aufnahme von Geschirr (15) oder Besteck trägt, wobei eine Mehrzahl von Trägerelementen (11) von der Basisstruktur (3) absteht,
**dadurch gekennzeichnet, dass**
die Mattenvorrichtung wenigstens ein Montageelement zur Befestigung der Mattenvorrichtung (1) im Korb (17) umfasst, insbesondere auf dem Boden des Korbes (17).

2. Mattenvorrichtung gemäß Anspruch 1, wobei die Mattenvorrichtung gegen Verrutschen innerhalb des Korbes während dessen Bewegung gesichert ist.

3. Mattenvorrichtung gemäß Anspruch 1 oder 2, wobei mindestens ein Montageelement mindestens ein Arretierungsmittel umfasst, insbesondere wenigstens ein Mittel zum Klemmen der Mattenvorrichtung an einen Draht eines Korbes (17) einer Geschirrspülmaschine.

4. Mattenvorrichtung gemäß einem der Ansprüche 1 bis 3, wobei wenigstens ein Montageelement an der Basisstruktur (3) angeordnet ist und geeignet ist, die Mattenvorrichtung (1) am Boden des Korbes (17) zu befestigen.

5. Mattenvorrichtung gemäß einem der Ansprüche 1 bis 4, wobei die Trägerelemente (11) Stifte, Bolzen, Finger, Tellereinsätze und/oder Tasseneinsätze aufweisen.

6. Mattenvorrichtung gemäß einem der Ansprüche 1 bis 5, wobei die Trägerelemente (11) flexibel und/oder elastisch sind.

7. Mattenvorrichtung gemäß einem der Ansprüche 1 bis 6, wobei die Trägerelemente (11) ein weiches und/oder ein steifes und/oder ein halbsteifes Material umfassen.

8. Mattenvorrichtung gemäß einem der Ansprüche 1 bis 7, wobei die Trägerelemente (11) zwei Komponenten umfassen, die wenigstens eine weiche Komponente enthalten.

9. Mattenvorrichtung gemäß einem der Ansprüche 1 bis 8, wobei mindestens ein Teil der Trägerelemente (11) zumindest teilweise hohl ist, insbesondere mindestens ein Teil der Trägerelemente eine Vertiefung von unten aufweist.

10. Mattenvorrichtung gemäß einem der vorangehenden Ansprüche, wobei die Basisstruktur (3) eine netzartige Form aufweist.

11. Mattenvorrichtung gemäß einem der vorangehenden Ansprüche, wobei die Basisstruktur (3) flexible Verbindungselemente (7, 9) aufweist, die untereinander verbindbare oder untereinander verknüpfbare Vereinigungselemente der Basisstruktur (3) sind.

12. Mattenvorrichtung gemäß Anspruch 11, wobei die Vereinigungselemente Knotenelemente sind und jedes flexible Verbindungselement (7, 9) wenigstens zwei Knotenelemente miteinander verknüpft.

13. Mattenvorrichtung gemäß einem der vorangehenden Ansprüche, wobei die Mattenvorrichtung (1) wenigstens teilweise aus elastischem Material gebildet ist.

14. Mattenvorrichtung gemäß Anspruch 13 in Abhängigkeit von Anspruch 1, wobei wenigstens die Basisstruktur (3) aus elastischem Material gebildet ist.

15. Mattenvorrichtung gemäß einem der vorangehenden Ansprüche, wobei die Basisstruktur (3) und/oder die Trägerelemente (11) aus einem flexiblen Material und/oder einem halbsteifen Material zusammengesetzt sind.

16. Mattenvorrichtung gemäß einem der vorangehenden Ansprüche, wobei das flexible Material EPDM, Sandopren oder ein weiches Kunststoffmaterial ist.

17. Mattenvorrichtung gemäß Anspruch 15 oder 16, wobei das halbsteife Material ein insbesondere mit einem elastischen Material beschichteter, dünner Metalldraht oder ein Kunststoffmaterial ist,

18. Mattenvorrichtung gemäß einem der vorangehenden Ansprüche, wobei die Breite der Mattenvorrichtung (1) kleiner als 30 c, insbesondere kleiner als 25 cm, vorzugsweise kleiner oder gleich 20 cm, ist.

19. Mattenvorrichtung gemäß einem der vorangehenden Ansprüche, wobei die Tiefe der Mattenvorrichtung (1) kleiner als 30 cm, insbesondere kleiner als 25 cm, vorzugsweise kleiner oder gleich 20 cm, ist.

20. Geschirrspülmaschine oder Korb (17) einer Geschirrspülmaschine, umfassend eine Mattenvorrichtung (1) gemäß einem der vorangehenden Ansprüche.

## Revendications

1. Dispositif formant tapis apte à être agencé dans un panier (17), en particulier sur le fond d'un panier (17), d'un lave-vaisselle, le dispositif formant tapis comprenant une structure de base (3) comprenant au moins partiellement une structure de support rigide qui est au moins partiellement recouverte par un matériau élastique et/ou supporte une structure flexible (11) pour recevoir des assiettes (15) ou des couverts, où une pluralité d'éléments de support (11) font saillie de la structure de base (3), **caractérisé en ce que** le dispositif formant tapis comprend au moins un élément de montage pour monter le dispositif formant tapis (1) dans le panier (17), en particulier sur le fond du panier (17).

2. Dispositif formant tapis selon la revendication 1, dans lequel le dispositif formant tapis est protégé contre un coulissement dans le panier durant son mouvement.

3. Dispositif formant tapis selon la revendication 1 ou 2, dans lequel au moins un élément de montage comprend au moins un moyen de positionnement, en particulier au moins un moyen pour attacher le dispositif formant tapis à un fil d'un panier (17) d'un lave-vaisselle.

4. Dispositif formant tapis selon l'une quelconque des revendications 1 à 3, dans lequel au moins un élément de montage est agencé à la structure de base (3) apte à monter le dispositif formant tapis (1) sur le fond d'un panier (17).

5. Dispositif formant tapis selon l'une quelconque des revendications 1 à 4, dans lequel les éléments de support (11) comprennent des axes, tenons, doigts, inserts de plaque et/ou inserts de coupelle.

6. Dispositif formant tapis selon l'une quelconque des revendications 1 à 5, dans lequel les éléments de support (11) sont flexibles et/ou élastiques.

7. Dispositif formant tapis selon l'une quelconque des revendications 1 à 6, dans lequel les éléments de support (11) comprennent un matériau mou et/ou rigide et/ou semi-rigide.

8. Dispositif formant tapis selon l'une quelconque des revendications 1 à 7, dans lequel les éléments de support (11) comprennent deux composants incluant au moins un composant mou.

9. Dispositif formant tapis selon l'une quelconque des revendications 1 à 8, dans lequel au moins une portion des éléments de support (11) est au moins partiellement creuse, en particulier au moins une portion de l'élément de support possède un évidement depuis en dessous.

10. Dispositif formant tapis selon l'une quelconque des revendications précédentes, dans lequel la structure de base (3) a une forme configurée en bande.

11. Dispositif formant tapis selon l'une quelconque des revendications précédentes, dans lequel la structure de base (3) possède des éléments de connection flexibles (7, 9) qui sont des éléments de jonction pouvant être connectés ou reliés entre eux de la structure de base (3).

12. Dispositif formant tapis selon la revendication 11, dans lequel les éléments de jonction sont des éléments de noeud, et chaque élément de connection flexible (7, 9) établit une liaison entre au moins deux éléments de noeud.

13. Dispositif formant tapis selon l'une quelconque des revendications précédentes, dans lequel le dispositif
formant tapis (1) est au moins partiellement réalisé en matériau élastique.

14. Dispositif formant tapis selon la revendication 13 dépendant de la revendication 1, dans lequel au moins la structure de base (3) est réalisée en matériau élastique.

15. Dispositif formant tapis selon l'une quelconque des revendications précédentes, dans lequel la structure de base (3) et/ou les éléments de support (11) sont réalisés en un matériau flexible et/ou un matériau semi-rigide.

16. Dispositif formant tapis selon l'une quelconque des revendications précédentes, dans lequel le matériau flexible est le EPDM, Sandoprène ou un matériau plastique mou.

17. Dispositif formant tapis selon la revendication 15 ou 16, dans lequel le matériau semi-rigide est un fil métallique mince ou un matériau plastique, en particulier revêtu d'un matériau élastique.

18. Dispositif formant tapis selon l'une quelconque des revendications précédentes, dans lequel la largeur du dispositif formant tapis (1) est inférieure à 30 cm, en particulier inférieure à 25 cm, de préférence inférieure ou égale à 20 cm.

19. Dispositif formant tapis selon l'une quelconque des revendications précédentes, dans lequel la profondeur du dispositif formant tapis (1) est inférieure à 30 cm, en particulier inférieure à 25 cm, de préférence inférieure ou égale à 20 cm.

20. Lave-vaisselle ou panier (17) d'un lave-vaisselle comprenant un dispositif formant tapis (1) selon l'une quelconque des revendications précédentes.
